# EUROPEAN PATENT APPLICATION

(11) **EP 4 527 420 A1**
(43) Date of publication of application: **26.03.2025**
(21) Application number: 24166810.2
(22) Date of filing: 27.03.2024
(51) Int. Cl.: A61L 27/22, A61L 27/50, A61L 27/52

(54) **METHOD FOR PREPARING A HIGH-TOUGHNESS SILK FIBROIN HYDROGEL AS ARTIFICIAL TENDON/LIGAMENT**

(30) Priority: 22.09.2023 CN 202311241159
(71) Applicant: Liangzhu Laboratory, Hangzhou city Zhejiang Province 311113 (CN); Zhejiang University, Hangzhou, Zhejiang 310058 (CN)
(72) Inventor: SHEN, Weiliang, Hangzhou city, 310058 (CN); HUANG, Wenwen, Hangzhou city, 310058 (CN); OUYANG, Hongwei, Hangzhou city, 310058 (CN); NIE, Kexin, Hangzhou city, 310058 (CN); ZHOU, Sicheng, Hangzhou city, 310058 (CN); WU, Boxuan, Hangzhou city, 310058 (CN); YE, Jinchun, Hangzhou city, 310058 (CN)
(74) Representative: Piotrowicz, Pawel Jan Andrzej

(57) **Abstract**

The present invention provides a preparation method for a high-strength and high-biocompatibility silk fibroin hydrogel scaffold with a biomimetic microstructure for repair and reconstruction of tendons/ligaments. The preparation method is characterized in that a natural silk fibroin material is processed through directional freezing and coaxial hot stretching, where a heating temperature needs to be higher than a glass transition temperature of the silk fibroin material, such that a maximum tensile strength of the silk fibroin hydrogel scaffold increases, a microscopic directional pore structure is formed, and finally a mechanically reinforced hydrogel scaffold with good biocompatibility is obtained. An artificial tendon/ligament (rotator cuff, anterior/posterior cruciate ligament, medial/lateral collateral ligament, medial patellar retinaculum, patellar ligament, Achilles tendon, and the like) scaffold prepared by using the hydrogel scaffold can reach the mechanical strength similar to that of the tendons/ligaments in the human body and has good biocompatibility, and a microstructure of the scaffold is provided with micron-scale directional pores, helping cells to directionally grow on a surface of the material and inside the material, which can achieve high repair efficiency.

## Description

### CROSS-REFERENCE TO RELATED APPLICATIONS

This application claims priority to Chinese Patent Application No. 2023112411598, filed on September 22, 2023, and all the applied contents including but not limited to the specification, abstract, claims, and accompanying drawings of this application are incorporated by reference in their entirety as a part of this application.

### BACKGROUND OF THE INVENTION

### Field of the Invention

The present invention relates to the technical field of hydrogel preparation, specifically relates to a preparation method for a high-toughness silk fibroin hydrogel, and in particular to a preparation method for a high-toughness silk fibroin hydrogel as rotator cuff, anterior/posterior cruciate ligament, medial retinaculum substitute, and the like.

### Description of the Related Art

The tendon/ligament injury is one of the common sports injuries. The tendon and ligament of the human body are mainly composed of dense type I collagen proteins and are hard to heal after injury due to a relatively low cell content and a relatively weak angiogenic ability. For example, a patient with a huge rotator cuff tear, an anterior cruciate ligament tear, and other large-scale tendon and ligament injuries usually needs to adopt patches for adjuvant treatment. A lot of preparation methods for a tendon/ligament repair material are available at present, and the traditional technology, such as an autologous material and an allogeneic material, has issues, including insufficient sources, easily causing complications at donor sites, rejection reactions, and disease propagation; and currently, a small number of artificial materials are used in the clinic, such as a French ligament advanced reinforcement system (LARS) ligament, Chinese "Ligatech" and the like, which exist issues of low strain, poor biocompatibility, difficultly integrating with host tissues and the like, easily leading to bone tunnel loosening, fatigue, and fracture, etc. These disadvantages lead to a poor repair effect for tendon/ligament injury, and searching for a scaffold material with both outstanding mechanical properties and biocompatibility has become a research hotspot.

Silk fibroin is widely applied in preparing tissue engineering scaffolds and has better biocompatibility, degradability, and excellent mechanical properties. The silk fibroin has a rich source and may be extracted from cocoon silk. The silk has a unique protein polymer structure, takes the silk fibroin as a kernel, and has a surface adhered with a sericin protein. The silk fibroin may be obtained by degumming the silk. The silk fibroin contains 18 amino acids, most of which are glycine, alanine, and serine. The silk fibroin may be completely degraded in vivo, during its degradation, plenty of amino acids, especially proline and lysine, will enrich in a cell microenvironment, and these two amino acids are the main raw materials for collagen synthesis. The repeated GAGAG sequence in the silk fibroin can form a secondary structure β-pleated sheet in the form of self-assembly, and this structure gives the silk fibroin excellent mechanical strength and toughness. It is reported that the toughness of the silk fibroin can be even superior to that of the best synthetic material. At the same time, the silk fibroin has a controllable mechanical property. By adjusting the ratio of the β-pleated sheet in the silk fibroin, the mechanical strength of the silk fibroin material may be further improved and superior to most degradable polymeric materials commonly used at present, such as collagen and poly-polylactic acid. In addition to this, the surface of the silk material is easy to process and modify and may be processed in various forms, from a liquid-state solution to an organic solid, such as a gel, a pipe, a hydrogel, a micro balloon, and a film. These characteristics of the silk material enable the silk material to have a broad prospect in the application of tissue engineering.

However, the mechanical strength of the existing silk fibroin is still hard to reach and maintain the mechanical strength that is similar to the tendon/ligament in the human body, and therefore it is urgent to develop a novel silk fibroin scaffold with a better initial mechanical property and long-term maintenance.

### BRIEF SUMMARY OF THE INVENTION

To solve the problem, the present invention provides a preparation method for a high-toughness silk fibroin hydrogel as a tendon/ligament scaffold. The preparation method is characterized in that a natural silk fibroin material is processed through directional freezing and coaxial hot stretching, where a heating temperature needs to be higher than a glass transition temperature of the silk fibroin material, such that a maximum tensile strength of the silk fibroin hydrogel scaffold increases, a microscopic directional pore structure is formed, and finally a mechanically reinforced hydrogel material with good biocompatibility is obtained. The hydrogel material can be used for preparing the tendon/ligament scaffold, and the tendon/ligament scaffold can achieve mechanical strength similar to that of the tendons/ligaments (rotator cuff, cruciate ligament, Achilles tendon, and the like) in the human body and has good biocompatibility and biodegradability, and a microstructure of the scaffold is provided with micron-scale directional pores, helping cells to directionally grow on a surface of the material and inside the material, which can achieve high repair efficiency.

The present invention provides a preparation method for a hydrogel, and the method includes:
(1) performing directional freezing on a silk fibroin solution, such that a solvent in the silk fibroin solution changes from a liquid state to a solid state;
(2) enabling the solid-state solvent in a product obtained in Step (1) to be sublimated and dried; and
(3) performing hot stretching on a freeze-dried product to obtain the hydrogel.

In some embodiments, there are many sublimation methods in Step 2, and any method that enables the solvent to directly change from the solid state to a gaseous state is feasible. Different sublimation methods can be selected based on the properties of the solvent, such as freeze-drying. When the solvent is water, a frozen ice block can be directly placed into a freeze dryer and freeze-dried, such that the water can be directly vaporized or sublimated from a block of solid-state ice to be volatilized, leaving set silk fibroin. In some embodiments, the solvent is completely volatilized, and the sublimation here can indicate 100% volatilization of the solvent, or 99%-80% of the solvent can be directly sublimated from the solid state to the gaseous state.

In some embodiments, the solvent in the silk fibroin solution includes water, such as deionized water, purified water, mineral water, and any water molecule in nature. Of course, the solvent is not water but can be any other solvent that can dissolve silk fibroin, as long as such solvents can become a solid state at a low temperature.

In some embodiments, the silk fibroin solution described in the present invention is made from degummed natural silk. It can be understood that, in some embodiments, the silk fibroin made from degummed natural silk can be directly purchased. In some embodiments, synthetic silk fibroin may also be employed in this application.

"Directional freezing," as defined in the present invention, is placing a polymer solution in a temperature field for cooling. As the temperature decreases, the solvent (usually water) is gradually solidified into ice crystal columns along a direction where the temperature gradually decreases in a gradient-type way, and polymer chains are extruded, directionally rearranged, and embedded among the ice crystal columns. Generally, a cylindrical or square container (chamber) is placed on or in contact with a freezing source, and the chamber is filled with the silk fibroin solution; the silk fibroin solution is directionally and gradually solidified from a direction approaching the freezing source to a direction departing from the freezing source; and the ice crystal columns are progressively formed along the direction. In other words, during freezing, the solvent in the solution is gradually frozen and solidified, and gradually changes from the liquid state to the solid state, and the ice crystal columns (if the solvent is water) are formed along the direction of gradual freezing. In this process, as an orientation template of the polymer chains, the ice crystals have the function of physical confinement, thereby playing an orientation role. The term "gradually" in the present invention is a relative concept. When a specified volume of a solution contact with the freezing source, the solution changes from a liquid state to a solid state from a place approaching the freezing source, and then the solution is gradually solidified from a direction approaching the freezing source to a direction departing from the freezing source as time goes by until the solution entirely becomes the solid state. In this process, the structure of the ice crystals is gradually formed and extended as time goes by, the extension direction thereof is substantially the same as the solidification direction thereof.

The "directional freezing" herein can also be understood as that when the solution approaches a temperature (only with one solidification temperature source) environment where the solvent can be solidified, the solvent in the solution is gradually solidified from the liquid state to the solid state from a direction approaching the solidification temperature source to a direction departing from the solidification temperature source. Such solidification has directionality; in a case that the solidification temperature is a single temperature source, the solidification direction is unidirectional; when the solution is between two solidification temperature sources, the solvent in the solution is gradually solidified from both ends to the middle, and the solidification directions are opposite. Actually, if the solidification temperature source is located in the middle of the solvent, the solvent is gradually solidified from the middle thereof to the outside in a direction where the solvent is dispersed from the middle thereof to both ends thereof. The above directions, regardless of single direction, two opposite directions, or divergent directions, are specific embodiments of directional freezing and solidification. In some embodiments, directional freezing is to change a solvent for dissolving silk fibroin from the liquid state to the solid state, for example, to change the water from the liquid state to the solid state; for example, the water exists in the form of ice.

Of course, the directional freezing herein does not include such a way, that is, the silk fibroin solution is allowed to be placed in a three-dimensional surrounding freezing environment, for example, all the solution is placed in a freezing refrigerator; the ambient temperature , wich is around to the solution, thereof is lower than the solidification temperature of the solvent; although this can also change the solvent from the liquid state to the solid state, this is not the meaning of "directional freezing" described in the present invention.

Therefore, anisotropic structures in different directions can be formed through directional freezing, such that materials are spatially arranged in different directions, and the mechanical properties in the direction of directional freezing will be much stronger than those in the direction of non-directional freezing; in addition, as ice crystals or solid-phase solvents occupy the space, the local aggregation degree of silk fibroin molecules will increase, further enhancing the mechanical properties. Compared with ordinary silk fibroin hydrogels subjected to non-directional freezing, the maximum tensile stress of silk fibroin hydrogels subjected to directional freezing can be enhanced several times. Furthermore, by the directional freezing technology, the silk fibroin solution may form pipe structures arranged in parallel, which own parallel pores of 5-10um and are similar to the microstructure of the tendon. On the one hand, this microstructure may guide and stimulate the differentiation from tendon stem cells to tendon cells, and on the other hand, this microstructure is beneficial to the permeation, growth, and directional arrangement of the cells inside the scaffold, thus promoting the tendon regeneration efficiency and effect.

In some embodiments, for ease of specifically performing the directional freezing, the silk fibroin solution may be packed in a container, and then the solution in the container is subjected to directional freezing. For example, the container may be any shape, and the shape of the container may be any form, such as a sphere, cube, cuboid, or any other form. In some embodiments, the space inside the container is the cuboid; for example, the cross-section of the cuboid may be round, rhombus, square, oval, and other shapes. When the solution is injected or packed in the cuboid container, the solution is cooled by a freezing source from one end and gradually frozen and solidified to the other end from one end, thus gradually freezing in a longitudinal direction of the container, making the solvent change from a liquid state to a solid state in the longitudinal direction, thereby icing.

In some embodiments, the material is subjected to directional stretching in step (3). In some embodiments, the directional stretching shares a direction with the directional freezing. For example, if the silk fibroin solution is frozen and solidified in a single direction, the stretching direction is also a single stretching direction, which is substantially the same as the freezing direction. It may be understood that the directional freezing direction is a single direction (a single-arrow direction) from one end to the other end, while the stretching may be outward stretching (a double-arrow direction) from two points, and although the stretching directions are different, they are on the same line. For example, directional freezing is the gradual freezing and solidification along a longitudinal axis from one end to the other end in the cuboid container, while the stretching is also along the direction of the longitudinal axis, and the stretching carried out in two directions is along the direction of the longitudinal axis.

In some embodiments, the silk fibroin material is heated to a specific temperature while stretching. In some embodiments, the heating temperature is higher than the glass transition temperature of the silk fibroin material, and the mechanical properties of the material can be greatly improved through coaxial heating or directional stretching. The coaxial stretching with the directional freezing or the lower stretching with the directional freezing direction may give the material a certain strain, enabling the material to be stretched to a certain length; and the molecules in the material will be rearranged under the action of the generated tensile stress, thus the material owns a certain orientation structure in the stretching direction, which can further enhance the tensile strength in this orientation.

In addition, the amorphous polymer has three mechanical states, including a glassy state, a high-elastic state, and a viscous flow state. When the temperature is relatively low, the material is a rigid solid state that is similar to glass and can only occur a very small deformation under the action of an external force, and this state is the glassy state. When the temperature continues to rise to a certain scope, the deformation of the material increases obviously, and is relatively stable in a certain subsequent temperature interval, and this state is the high-elastic state. The glass transition temperature (Tg) refers to the corresponding temperature of the glassy state transformed into the high-elastic state. The silk fibroin is in a glassy state at a normal temperature or a room temperature (25 degree centigrade), and a relatively small deformation can occur only during coaxial stretching; however the heating enables the silk fibroin to be equal to or higher than Tg, the silk fibroin enters the high-elastic state, the silk fibroin molecule moves aggravatingly, the activity scope increases, the silk fibroin material of this state is easier to occur the deformation and may be stretched to a higher ratio during the coaxial or directional stretching, and the aggravation of the molecular movement is more conducive to the formation of orientation at the molecular level.

The stretching may be automatically completed by adopting the existing device, for example, the stretching is completed by adopting a high-temperature mechanical tester. The high-temperature mechanical tester is a scientific instrument used in the field of material science, mainly including an extensometer, a high-temperature furnace for heating and a rod and panel clamp for fixing the stretched material such as the silk fibroin and the silk fibroin material after directional freezing in the present invention. For example, all the devices introduced at this website may complete the stretching of the present invention. http://www.cxwannengsyj.com/html/2019/gaodiwen_0521/1596.html?sdclkid=ALos152NxSDibL-pA52G&bd_vid=10212208613159825381. ; https://www.zhongguoqingji.com/685cc91a-bdab-2768-53cf-5bde625d2d7f/GDWWNSYJ.shtml?bd_vid=12561170255290571953;

The dried silk fibroin film has a glass transition temperature of about 178°C, and in a case that water molecules serve as plasticizers, the conditions adopted by the present invention may enable the glass transition temperature to reduce by 77°C, which greatly simplifies the operation difficulty and reduces the cost.

Therefore, in some embodiments, the silk fibroin of directional freezing is frozen and dried in the present invention, allowing the solidified solvent to gasify and evaporate to only retain the silk fibroin itself, and then cross-linking and/or hydration and directional stretching may be carried out at a relatively low temperature, which simplifies the operation difficulty and reduces the cost. In the present invention, the hydrated silk fibroin is subjected to coaxial stretching with different temperatures above Tg and different stretching strains, such that the maximum stretching stress of the silk fibroin hydrogel is improved by about 10 times. The directional freezing and hot stretching adopted by the present invention also have significant synergy, such that the maximum tensile strength of the silk fibroin hydrogel is improved by nearly 100 times, and at the same time the silk fibroin hydrogel also has better biocompatibility, and the microscopic directional pore structure helps the cell ingrowth and is more conducive to the repair of the tendon injury.

The hot stretching must be carried out after the directional freezing, and the coaxial stretching must be carried out along the directional freezing direction: 1. The directional freezing step is the precondition for the hot stretching, and the direct stretching without the directional freezing will lead to the direct fracture of the silk fibroin hydrogel; and 2. If the coaxial stretching is not carried out according to the directional freezing direction after the directional freezing, the direct fracture of the silk fibroin hydrogel will also be caused.

Further, the preparation method includes the following steps:
(1) Pouring the silk fibroin solution into a mold for directional freezing, to obtain a silk fibroin ice block;
(2) Freezing, drying and cross-linking the silk fibroin ice block, soaking the silk fibroin ice block in water for hydration, to obtain the hydrogel; and
(3) Hot stretching the hydrogel.

In some embodiments, the hot stretching shares a direction with the directional freezing.

Further, the directional freezing in Step (1) means that the freezing source is close to the mold, enabling the silk fibroin solution in the mold to directly generate an ice crystal in a direction from a contact surface approaching to the freezing source to a contact surface away from the freezing source. In some embodiments, the temperature of the freezing source is lower than -20°C, -10°C, -5°C, -2°C or -1°C.

Any apparatus or product with a cooling effect may serve as the freezing source of the present invention, or in any case, the solvent in the silk fibroin solution may serve as the freezing source for solidification, for example, different temperature freezing sources may be set with the different solidifying point temperatures of the solvent. The "freezing source" set in the present invention changes by replying on the solvent for dissolving the silk fibroin and also changes with the environment condition, and the freezing and solidifying temperatures of the solvent are also different due to different solvents and different environments. For example, when the solvent is water, the ice point of the water decreases along the increase of the barometric pressure, such that the water solidifying temperature decreases below 0°C, and of course, the temperature is lower, and the water solidifying time is shorter. In addition, the solution has a certain height, for example, the solution has a height of 1 mm-200 cm, enabling the whole solution to be solidified completely, and the required time is also different; for example, in a case that the solvent is water and the thickness is 1 mm, the solidification may be achieved below 0°C, and when the thickness is 10 cm or 20cm, a lower temperature or a longer time may be required. It can be understood that those of ordinary skill in the art can make an arbitrary selection by reading the summary of the present invention and according to actual situations, and finally the silk fibroin solution achieves solidification.

In some embodiments, a dynamic source may be achieved by adopting liquid nitrogen. In some embodiments, one end of one metal element is connected with the liquid nitrogen while the other end is connected with one end of the container containing the silk fibroin solution during directional freezing, and thus the silk fibroin solution may achieve the directional freezing. In some embodiments, in Step (1), the silk fibroin solution is poured into a columnar space of the mold, and the mold is placed above a metal rod, of which one end is soaked in liquid nitrogen.

In some embodiments, the metal rod serving the freezing source is located below the mold and in complete contact with the bottom surface of the module, the ice crystal of the silk fibroin material will grow up from the bottom cold source during freezing, thus having a certain directivity, this direction is perpendicular to the bottom surface that is in contact with the freezing source, and then growth is carried out in a direction away from the freezing source.

In some embodiments, the metal rod serving as the freezing source is located on the side of the mold, and in complete contact with the side of the mold, the ice crystal of the silk fibroin material will start the directional growth from the side that is in contact with the freezing source during freezing, the growth direction of the ice crystal is perpendicular to the bottom surface of the freezing source, and then growth is carried out in a direction away from the freezing source.

In some embodiments, the metal rod serving as the freezing source is located on the top surface of the mold, and in complete contact with the top surface of the mold, the ice crystal of the silk fibroin material will start the downward growth from the top surface that is in contact with the freezing source during freezing, the growth direction of the ice crystal is perpendicular to the bottom surface of the freezing source, and then growth is carried out in a direction away from the freezing source.

In some embodiments, in Step (1), the silk fibroin solution is poured into the mold, in which a columnar space of 4*4*20 mm is provided. The mold is placed above one aluminum rod, the width of the aluminum rod may completely cover the contact surface with the mold, and one side of the aluminum rod is soaked in liquid nitrogen. After 10 minutes, the mold is removed, and a silk fibroin ice block is removed from the mold and placed in a refrigerator at -80°C for the night.

Further, the silk fibroin is cross-linked after directional freezing and drying. In some embodiments, for example, the cross-linking in Step (2) is chemical cross-linking or enzyme cross-linking.

Since the silk fibroin can be dissolved in water, it also needs to be cross-inked to form the hydrogel, and by cross-linking, on the one hand, the silk fibroin scaffold may keep stable in the water solution, and on the other hand, the material strength may also be improved further.

It is to be noted that the cross-linking treatment must be carried out after the directional freezing is completed because the molecular structure is fixed in advance if the cross-linking is performed first, the directional arrangement along the growth of the ice crystal during the directional freezing cannot be performed, and the microscopic directional structure cannot be formed, thus the scaffold will lose the directional structure eventually.

Any method may be adopted for cross-linking in the present invention, such as ethanol cross-linking, other chemical methods of cross-linking, and enzyme cross-linking, and the cross-linking capable of implementing the silk fibroin material can be used for the present invention and fall in the protection scope of the present invention.

Further, in Step (2), the silk fibroin ice block is placed in a freeze dryer and freeze-dried, soaked in the anhydrous ethanol to be cross-linked, and soaked in the water to be fully hydrated.

In some embodiments, anhydrous ethanol is adopted in the present invention for cross-linking, before cross-linking, the silk fibroin ice block needs to be freeze-dried first, in a case that the solvent is water, the ice block is gasified to remove the ice block and keep the directional silk fibroin, this is because if the direct cross-linking without freeze-drying is carried out and the low-temperature silk fibroin ice block meets the relatively high temperature ethanol, the microstructure therein will be damaged with the melting of the ice crystal before the ethanol is cross-linked. However, after the silk fibroin ice block is freeze-dried, the complete silk fibroin scaffold with the microscopic directional structure is obtained, and then placed into the anhydrous ethanol for intermolecular cross-linking, to form the more stable silk fibroin hydrogel structure.

In some embodiments, after being cross-linked, the silk fibroin ice block may also be soaked in the water to be hydrated, this is because the dried silk fibroin material has a glass transition temperature of about 178°C, in a case that the humidity changes and the water molecules serve as the plasticizers, the glass transition temperature may be reduced to below 77°C, and reducing the glass transition temperature greatly simplifies the operation difficulty of the hot stretching and reduces the cost; and in addition, the coaxial stretching is carried out in a case of higher than 100°C, the moisture in the hydrogel will be evaporated quickly, leading to the loss of the water molecules as the plasticizers, and making the hydrogel become brittle and have a limited stretching length. Therefore, the hydrated silk fibroin may be below 100°C, for example, 95°C, 85°C, 75°C, 65°C, 55°C, 45°C, 35°C, 25°C and 15°C, preferably below 77°C. Full hydration means that the silk fibroin is soaked in the water until the scaffold quality is basically unchanged, indicating that the hydration is full, and the silk fibroin is usually soaked for 48 h at least.

In some embodiments, the silk fibroin ice block is placed in a freeze dryer and freeze-dried for 72 h to obtain a dried scaffold, the silk fibroin scaffold is soaked in the anhydrous ethanol to be cross-linked for 6 h, and soaked in the water for 48 h to be fully hydrated.

Further, the hot stretching in Step (3) indicates heating up the product to above a glass transition temperature of the silk fibroin and then performing coaxial stretching on the product, where the coaxial stretching shares a direction with the directional freezing.

The coaxial stretching shares a direction with the directional freezing, such that the prepared scaffold has a better mechanical property, and the microscopic directional pore exists, which is beneficial for cell ingrowth.

After directional freezing, drying, and cross-linking, the hydrogel has vertical stripes in the appearance, and the microscopic pore direction formed by the directional freezing may be identified through the naked eye. At this time, heating is carried out again to above the glass transition temperature of the silk fibroin, and then the directional coaxial stretching is performed in the same direction, to achieve the rapid increase of the mechanical strength.

Further, in Step (3), the hydrated hydrogel is preheated at a temperature of 25°C to 130°C, and then stretched to 30% to 90% of an initial length.

In some embodiments, the heating temperature is only above the glass transition temperature of the silk fibroin, and the glass transition temperature of the hydrated silk fibroin hydrogel may be reduced to below 77°C, such as 25°C.

Further, in Step (3), the hydrated hydrogel is preheated for 5 min at a temperature of 25°C to 130°C, and then stretched to 30% to 90% of an initial length and kept for 15 min; after stretching, the material is removed and soaked into the water to be hydrated to obtain a finished hydrogel.

In some embodiments, the hot stretching may be completed in a hot stretching device, such as the high-temperature mechanical tester, as long as the hot stretching can be achieved at the same time.

In some embodiments, the pretension force for hot stretching can pull the hydrogel, such that the hydrogel has no obvious shrinking under the pretension force.

In some embodiments, the ratio of the hot stretching is not fixed, the mechanical property of the hydrogel may be improved through stretching, and therefore the stretching may be carried out in a ratio according to actual needs, to ensure that the scaffold will not be pulled to fracture. The initial length of the stretching is above 90%, and some fractures may occur to the material.

In some embodiments, in Step (3), the hydrated hydrogel is given a pretension force (2N), and preheated for 5 min at 95°C; then, the material is stretched to 30% to 90% of the initial length at a rate of 10 mm/min below 95°C and kept for 15 min; after stretching, the material is removed and soaked into the water to be hydrated to obtain a finished hydrogel.

Further, the silk fibroin solution in Step (1) is prepared by: removing silkworm chrysalis from silkworm cocoons to obtain a silk fibroin fiber, boiling the silk fibroin fiber with 0.02M sodium carbonate for 30 min and degumming the same, drying the degummed silk fibroin fiber in a fume hood for 12 h, dissolving the silk fibroin fiber in 9.3M lithium bromide at 60°C for 4 h, dialyzing the solution with a dialysis bag for 72 h to remove the lithium bromide; and naturally air-drying the obtained solution in the fume hood for 6 h to obtain a silk fibroin solution with a concentration of 15%.

The natural silk is used in the present invention and prepared into the silk fibroin solution after degumming treatment, the silk fibroin solution is subjected to directional freezing and freeze-dried, and then the cross-linking is carried out by using the anhydrous ethanol, after fully hydrated, the obtained scaffold is stretched to 60% of the initial length at a speed of 10 mm/min in a high-temperature condition (95°C) higher than the glass transition temperature, and kept for 15 min; after removed, the scaffold is hydrated gain to obtain a finished hydrogel.

According to another aspect, the present invention provides use of the method in improving the mechanical properties of the material, the method is that the first is subjected to directional freezing and freeze-dried first, then subjected to the coaxial hot stretching, the heating temperature is higher than the glass transition temperature of the material, and the material is polyvinyl acetate (PVA), gelatin and the like.

The present invention provides a synergistic processing method for hot stretching and directional freezing. The directional freezing can not only give the material the directional pore structure at the microscale but also improve the mechanical property of the material through the formation of various anisotropic structures. The material after the directional freezing is further subjected to the hot stretching above the glass transition temperature, which may further improve the mechanical property of the material greatly and the maximum stretching stress of the material. Meanwhile, the hydrogel has micron-scale directional pores, which can, on the one hand, guide and stimulate the cell differentiation through a microscopic topology morphology, and on the other hand, help the infiltration of the cell on the scaffold, to quicken the tissue repairing.

The synergistic processing method for hot stretching and directional freezing provided by the present invention is not only suitable for the silk fibroin material, but also suitable for the improvement of the mechanical properties of other materials.

According to a yet aspect, the present invention provides the use of a silk fibroin hydrogel in preparing a rotator cuff patch or an anterior cruciate ligament substitution, the silk fibroin hydrogel is prepared by directional freezing and freeze-drying the silk fibroin solution as well as hot stretching, and the heating temperature is higher than the glass transition temperature of the silk fibroin.

In a word, the present invention plans to protect the processing method for stretching (hot stretching for short) above the glass transition temperature, and a synergistic processing method for hot stretching and directional freezing, for the improvement of the mechanical property of the material and the acquisition of the surface microstructure, which is beneficial for the tendon repairing efficiency.

The silk fibroin hydrogel scaffold provided by the present invention has the following beneficial effects:
1. Excellent mechanical property: the traditional silk fibroin hydrogel has a maximum stress of about 0.02-0.2 MPa, and the maximum stress in the present invention may reach above 10 MPa and is improved by hundreds of times;
2. The directional topological structure on the hydrogel surface may promote the directional arrangement of the tendon stem cells;
3. Better biocompatibility: the silk material itself has better biocompatibility, the organic reagent hard to remove is not used when preparing the hydrogel scaffold, such that the finally prepared scaffold has better biocompatibility;
4. Compared with the autologous tendon and the like, the silk material has a wide source and a simple preparation process; and
5. The synergistic processing method for hot stretching and directional freezing provided by the present invention is not only suitable for the silk fibroin material, but also suitable for the improvement of the mechanical properties of other materials.

### BRIEF DESCRIPTION OF THE DRAWINGS

FIG. 1A - FIG. 1C show a preparation flowchart of a silk fibroin hydrogel scaffold, where FIG. 1A is a schematic illustrative diagram of directional freezing, a conical structure in a cube shows a schematic diagram of an ice crystal structure, black dots are water molecules, and a curve between the water molecules shows a schematic diagram of silk fibroin; FIG. 1B is a schematic illustrative diagram of cross-linking and hydration; and FIG. 1C is a schematic illustrative diagram of directional stretching.
FIG. 2A is a schematic diagram of the operation of a mold filled with a silk fibroin solution being placed on an aluminum rod for directional freezing and one end of the aluminum rod being soaked in liquid nitrogen according to a specific embodiment of the present invention (Embodiment 1).
FIG. 2B is a schematic diagram of an operation structure of directional freezing according to a specific embodiment of the present invention.
FIG. 3 shows an SEM image of a hydrogel scaffold that is not subjected to directional freezing, and subjected to directional freezing and hot stretching after the scaffold is subjected to directional freezing in Embodiment 2, and it can be seen that a hydrogel not subjected to directional freezing presents an irregular pore structure under a microscope, and a hydrogel subjected to directional freezing has a directional pore structure under the microscope.
FIG. 4A is a comparison diagram of stretching curves of the hydrogel not subjected to directional freezing and the hydrogel subjected to directional freezing in Embodiment 2.
FIG. 4B is a comparison diagram of the mechanical properties of the hydrogel not subjected to directional freezing and the hydrogel subjected to directional freezing in Embodiment 2.
FIG. 5 shows a stress-strain curve of an ND₁₅ hydrogel that is subjected to a stretching test by using a universal mechanical tester in Embodiment 3.
FIG. 6 shows a stress-strain curve of the ND₁₅ hydrogel that is subjected to a fracture toughness test by using the universal mechanical tester in Embodiment 3.
FIG. 7 shows photos of a notched ND₁₅ hydrogel in a stretching process in Embodiment 3 (a leftmost photo shows the start of stretching, a middle photo shows a fracture in the stretching process, and a rightmost photo shows a result of a complete fracture in the stretching process).
FIG. 8A is a comparison diagram of stretching curves of silk fibroin hydrogels with different concentrations in Embodiment 4.
FIG. 8B is a comparison diagram of the mechanical properties of the silk fibroin hydrogels with different concentrations in Embodiment 4.
FIG. 9 is a DSC diagram of a silk fibroin hydrogel prepared by Step c in Embodiment 5.
FIG. 10A is a comparison diagram of stretching curves of silk fibroin hydrogels obtained by changing a heating temperature in Step d in Embodiment 5.
FIG. 10B is a comparison diagram of the mechanical properties of the silk fibroin hydrogels obtained by changing the heating temperature in Step d in Embodiment 5.
FIG. 11A is a comparison diagram of tensile curves of silk fibroin hydrogels obtained by changing a tensile ratio in Step d in Embodiment 6.
FIG. 11B is a comparison diagram of the mechanical properties of the silk fibroin hydrogels obtained by changing the tensile ratio in Step d in Embodiment 6.
FIG. 12A and FIG. 12B show tensile stress-strain curves of three groups of hydrogels (FIG. 12A), and maximum tensile stresses and Young's moduli (FIG. 12B) in Embodiment 7.
FIG. 13 is an HE staining diagram of a silk fibroin hydrogel as a lateral splint for repairing detached rabbit Achilles tendons in Embodiment 8.

### DETAILED DESCRIPTION OF THE INVENTION

The following further elaborates preferred embodiments of the present invention with reference to the accompanying drawings, and it should be noted that the embodiments described hereinafter are intended to facilitate the understanding of the present invention and do not impose any restriction on it. Raw materials and equipment used in the specific embodiments of the present invention are all known products and purchased from commercially available products.

### Embodiment 1 Preparation of silk fibroin hydrogel scaffold provided by the present invention

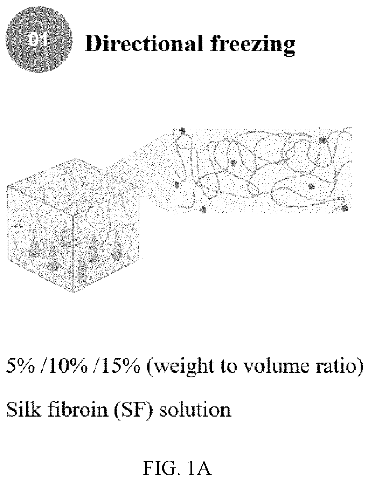

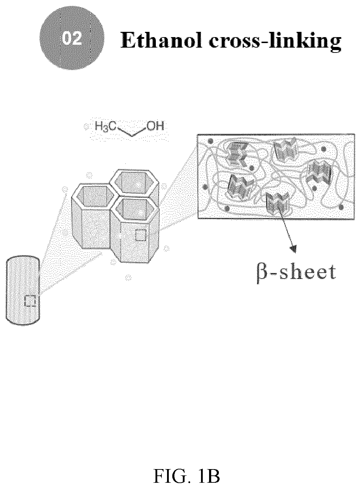

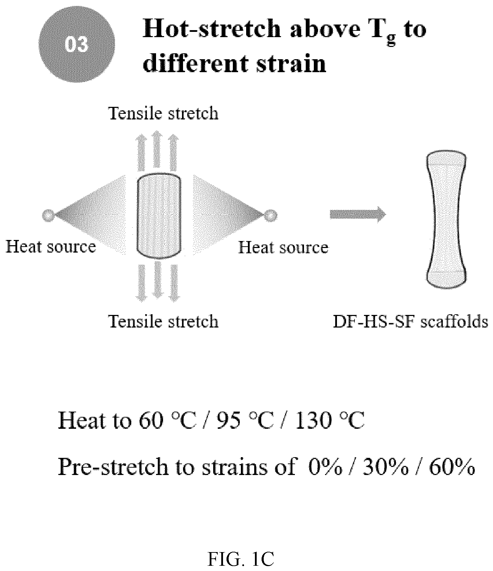

A preparation method for a silk fibroin hydrogel scaffold provided by this embodiment, as shown in FIG. 1, includes the following steps:
a. Preparation method of silk fibroin solution: silkworm cocoons (5g) were boiled with 0.02M sodium carbonate (2L) for 30 min; degummed silk fibroin fibers were dried in a fume hood for 12 h, and then dissolved in 9.3M lithium bromide at 60°C for 4h; a solution was dialyzed with a dialysis bag for 72 h to remove the lithium bromide; and the obtained solution was naturally air-dried in the fume hood for 6 h to obtain a silk fibroin solution with a concentration of 15%.
b. Directional freezing: the silk fibroin solution was poured into a 4 mm * 4 mm * 20 mm columnar space in a self-made mold, where the columnar space was provided with a cubic opening having 4 mm * 4 mm in size and 20 mm in depth, and fully filled with the silk fibroin solution; and the mold could be made of any metals, such as aluminum, iron, and alloy. The mold of this embodiment was made of an aluminum alloy material. Of course, in other examples, any space is possible; for example, cylinders, cubes, and cuboids had openings to realize freezing, such that the silk fibroin solution could flow into the space to realize directional freezing.

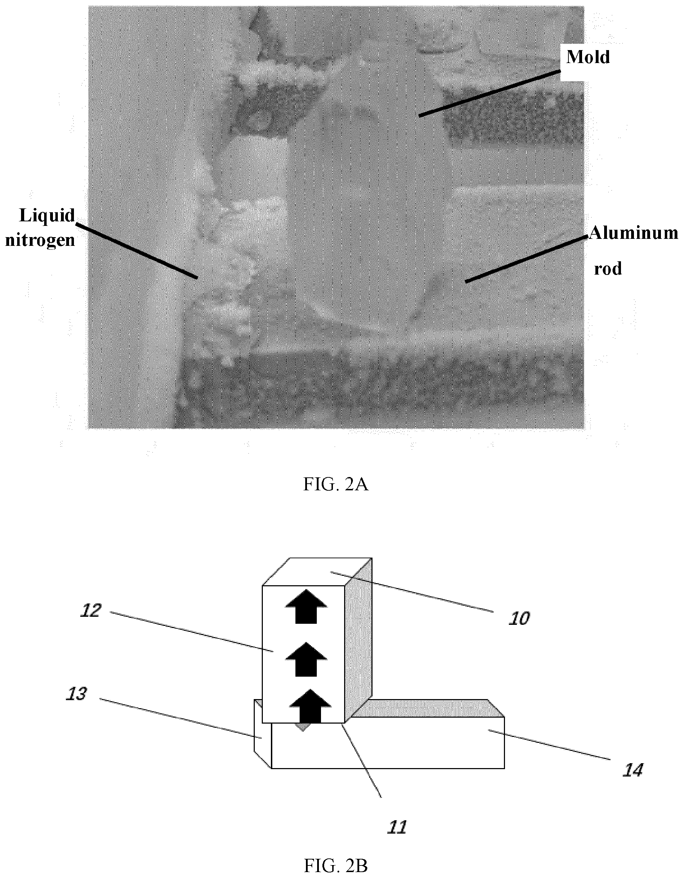

One end of an aluminum rod was immersed in liquid nitrogen (as shown in FIG. 2A), such that the aluminum rod was cooled by the liquid nitrogen to lower its temperature; the other end of the aluminum rod was placed into the mold filled with the silk fibroin solution (the bottom of the columnar space contacted with liquid), and the 4 mm * 4 mm bottom of the mold was in full contact with the surface of the aluminum rod; due to the heat transfer effect of the aluminum rod, the aluminum rod was gradually subjected to directional freezing from the bottom of the columnar space to the top thereof, such that the solution gradually changed into the solid state from the bottom, especially water gradually changed into the solid state from the bottom to the top, and formed ice crystals gradually extended from the bottom to an opening (as indicated by an arrow in Step 1 in FIG. 1A). After 10 minutes, the mold was removed, and a silk fibroin ice block was removed from the mold and placed in a refrigerator at -80°C for the night.

For example, referring to FIG. 2B, the opening 10 of the columnar space 12 was disposed in the upper surface of the columnar space, one end 13 of the aluminum rod could be located on the bottom 11 or the bottom 11 of the columnar space could be located on the surface of the aluminum rod; the other end 14 of the aluminum rod was in contact with the liquid nitrogen, such that the solution in the columnar space was gradually solidified from the bottom 11 to the opening 10, and the formed ice crystals also extended from the bottom to the opening, as shown by the arrow in fig. 2B for directional freezing.
c. Ethanol cross-linking: the silk fibroin ice block was placed in a freeze dryer and freeze-dried for 72 h, such that the ice block was vaporized and volatilized to obtain a dried scaffold, the silk fibroin scaffold was soaked in the anhydrous ethanol to be cross-linked for 6 h, and soaked in the water for 24 h to be fully hydrated.
d. Coaxial hot stretching: the hydrated hydrogel in Step C was clamped between clamps by a high-temperature mechanical tester (Model TSE, WANCE, model: ETM105D), and a pretension force (2N) was applied to the hydrated hydrogel; the hydrated hydrogel was preheated for 5 min at 95°C; then, at this temperature, the material was stretched to 60% of its initial length at a rate of 10 mm/min and kept for 15 min; and after heated up from its periphery to the clamps, the hydrogel was removed and soaked in the water for 24h to obtain a finished hydrogel. The stretching direction is the same as the freezing direction (as indicated by an arrow in FIG. 1C, stretching is performed towards two longitudinal ends), that is, the stretching is performed towards a longitudinal direction along a depth of 20 mm and may be understood as longitudinal stretching.

### Embodiment 2 Effect of directional freezing on silk fibroin hydrogel

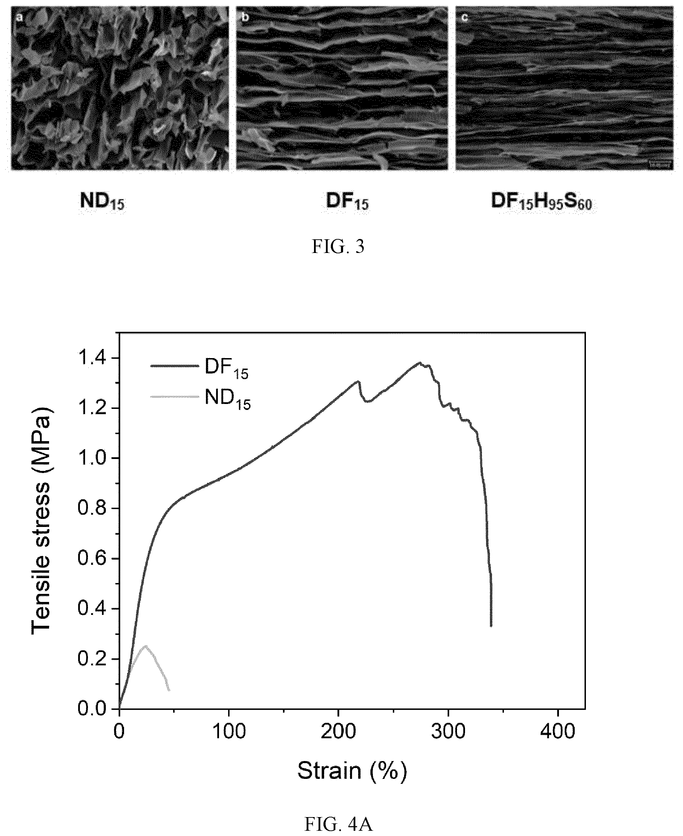

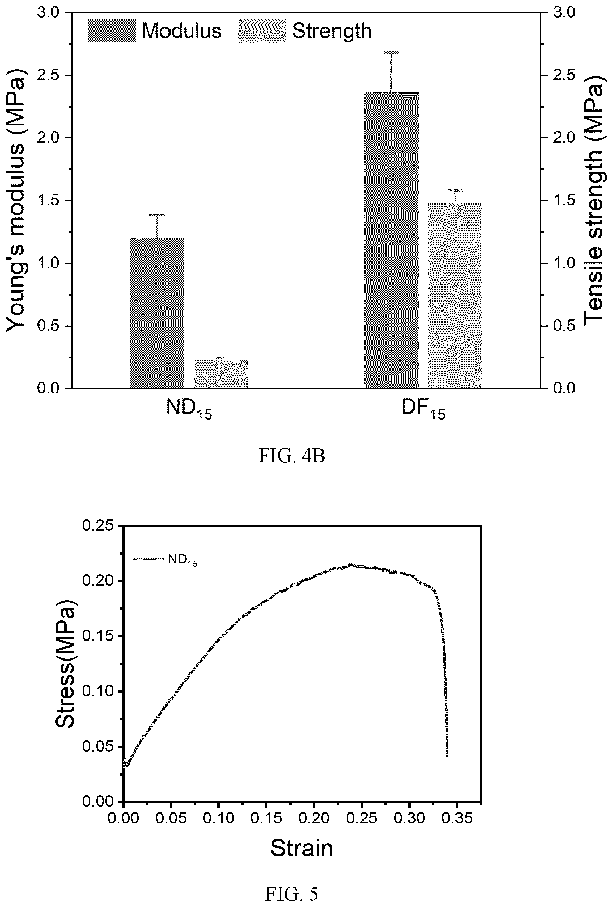

In this embodiment, two groups of silk fibroin solutions were taken respectively; one group of silk fibroin solutions was subjected to directional freezing by the method provided in Embodiment 1 and cross-linked with ethanol to prepare a directional silk fibroin hydrogel scaffold (DF); the other group of silk fibroin solutions was not subjected to directional freezing, and the solution prepared in Step a was poured into the mold and directly placed in a refrigerator at -80°C, freeze-dried and then cross-linked and hydrated with ethanol to form a non-directional silk fibroin hydrogel (ND). SEM images of two groups of hydrogel scaffolds prepared are shown in FIG. 3, where FIG. 3b (middlemost) shows a parallel pipe structure formed after directional freezing, and the parallel pipe structure has 1 µm to 10 µm parallel pores and is similar to a surface topology structure of tendons. FIG. 3a shows an SEM image of a non-directional hydrogel scaffold (leftmost), and the non-directional hydrogel scaffold is significantly different from the directional pipe structure as shown in FIG. 3b. The tensile curves for two groups of silk fibroin hydrogels are shown in FIG. 4A and FIG. 4B, where FIG. 4A shows a relationship diagram between a tensile ratio and a tensile stress, and FIG. 4B shows a comparison diagram between Young's modulus and tensile stress. DF₁₅ means DF prepared by silk fibroin with a concentration of 15%, and ND₁₅ means ND prepared by silk fibroin with a concentration of 15%. It can be clearly seen from FIG. 4 that the tensile stress and Young's modulus of silk fibroin prepared by directional freezing are obviously improved, where the tensile stress increases by about 6 times and the Young's modulus increases by about 1 times.

### Embodiment 3 Directional freezing step being a prerequisite for hot stretching

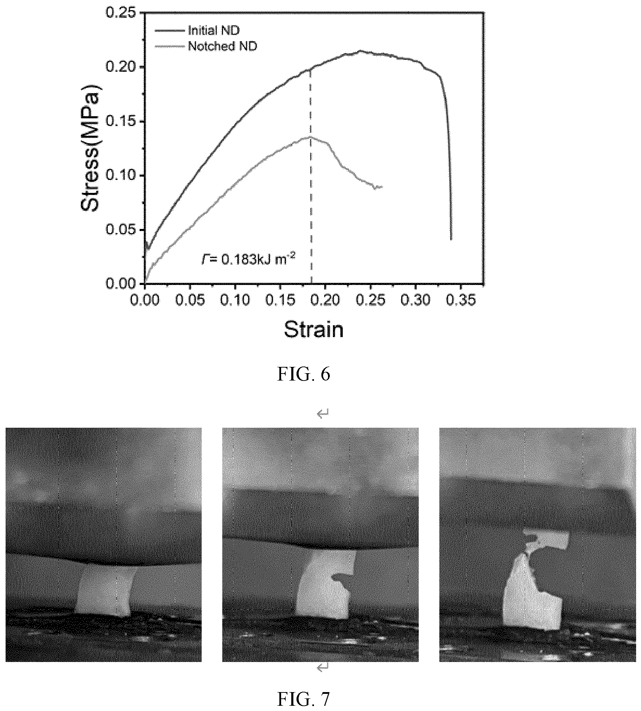

This embodiment only relates to a silk fibroin hydrogel. The silk fibroin hydrogel was prepared as follows: a solution prepared in Step a was poured into a mold and directly placed in a refrigerator at -80°C (non-directional), freeze-dried, and cross-linked with ethanol to form a non-directional silk fibroin hydrogel (ND₁₅). The mechanical properties of ND₁₅ hydrogels were tested by a universal mechanical tester, and a stress-strain curve as shown in FIG. 5 was obtained through a tensile test. As shown in FIG. 5, ND₁₅ has poor mechanical properties, with a maximum stress of only 0.21 MPa and a fracture strain of about 32%, showing low strength and poor toughness. In order to further test the fracture toughness of ND hydrogels, such hydrogels were subjected to a pure shear test. In the test, a pair of ND hydrogels, including an initial intact hydrogel (Initial ND) and a notched hydrogel (Notched ND), were tested at a time; and a stress-strain curve as shown in FIG. 6 was obtained through the stretching test for each pair of hydrogels, and the fracture toughness was obtained through calculation. FIG. 7 shows a photo of a notched ND₁₅ hydrogel in a stretching process. As shown in FIG. 7, the notch of ND₁₅ expands rapidly in the stretching process, causing the hydrogel to rupture rapidly under small strain (less than 20%). In combination with the calculated fracture toughness as shown in FIG. 6, the ND₁₅ hydrogel shows poor fracture toughness. In a word, the ND₁₅ hydrogel that is not subjected to directional freezing has poor mechanical strength and toughness and ruptures under small strain, so the hydrogel is not suitable for subsequent hot stretching tests. In other words, the directional freezing step is a prerequisite for hot stretching.

In addition, if a DF₁₅ hydrogel prepared through directional freezing is not subjected to directional stretching in a directional freezing direction, the hydrogel cannot obtain excellent mechanical properties and is difficult to stretch and easy to tear. For the DF₁₅ hydrogel prepared through directional freezing, the directional pipe structure determines that the pipes are easy to separate from each other; if the pipes are not hot-stretched in the directional freezing direction, the DF₁₅ hydrogel is easy to tear, such that a preparation process integrated with directional freezing and hot stretching cannot be successfully completed.

### Embodiment 4 Effect of silk fibroin concentration on silk fibroin hydrogel

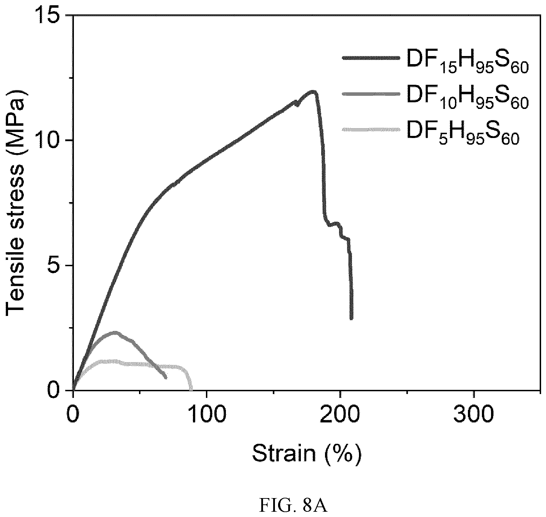

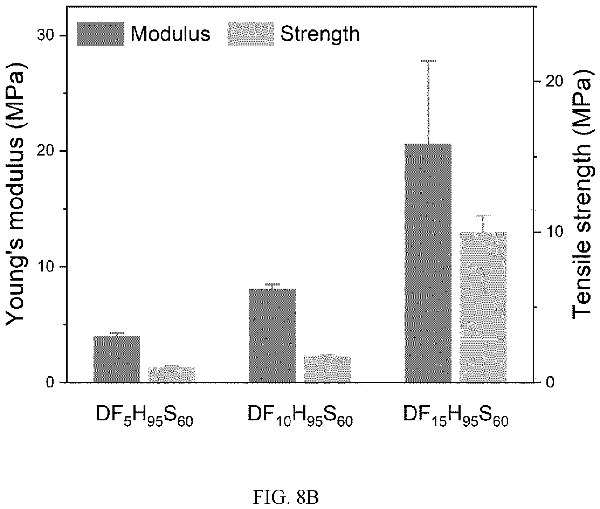

In this embodiment, four groups of silk fibroin solutions with concentrations of 5%, 10%, 15%, and 20% were taken respectively. After being subjected to directional freezing by the method provided in Embodiment 1, the silk fibroin solutions were subjected to coaxial directional stretching by 60% at 95°C to prepare a silk fibroin hydrogel scaffold. The tensile properties of two groups of hydrogel scaffolds were tested by a biomechanical tester. The tensile curves for silk fibroin hydrogels with different concentrations are shown in FIG. 8, where the left figure shows a relationship diagram between a tensile ratio and a tensile stress; and the right figure shows a comparison diagram between a Young's modulus and a tensile stress. DF₁₅H₉₅S₆₀ means that a silk fibroin hydrogel with a concentration of 15% is hot-stretched at 95°C at a tensile ratio of 60% to prepare a hydrogel. It can be seen from FIG. 8 that with the increase of the silk fibroin concentration, the scaffold has an increasingly obvious directional effect, with increasingly good tensile properties. Theoretically, the tensile properties of silk fibroin with a concentration of 20% or more will be further enhanced but will decrease due to its limited freeze-drying effect. Therefore, we determined 15% as a preferable concentration of silk fibroin.

### Embodiment 5 Effect of different temperatures of hot stretching on properties of silk fibroin hydrogel scaffold

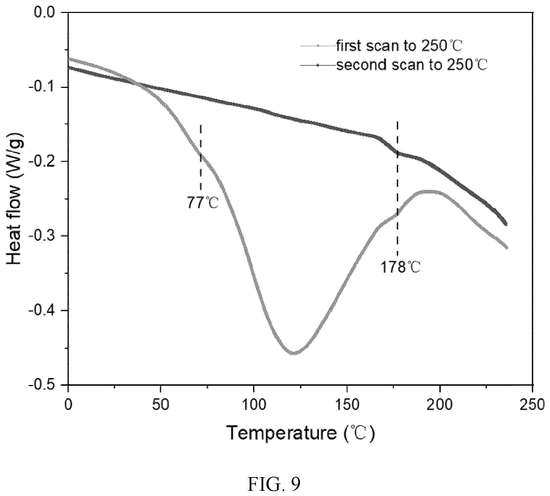

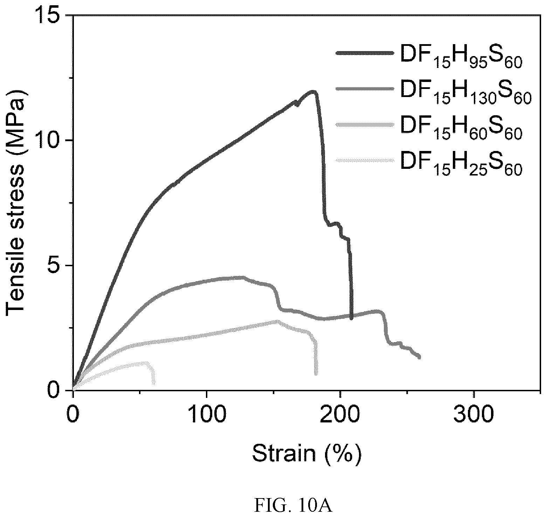

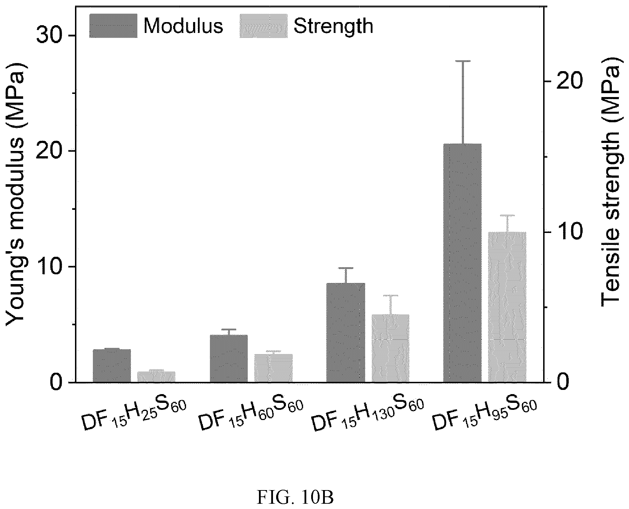

In this embodiment, a silk fibroin hydrogel was prepared by the method provided in Embodiment 1. Following Step c, the silk fibroin hydrogel was subjected to a DSC test, with test results as shown in FIG. 9. In the first heating process, a glass transition temperature of silk fibroin was about 77°C when water molecules served as plasticizers; in the second heating process, as the scaffold completely lost water, its plasticizing effect of water molecules was lost, and the glass transition temperature of the silk fibroin scaffold was elevated to 178°C. Therefore, in Step d, we set four temperatures, and stretching was performed by using different heating temperatures (25°C, 60°C, 95°C and 130°C); the maximum tensile stress of the prepared hydrogel scaffold was tested, and the mechanical properties of the hydrogel scaffold were tested by a biomechanical tester; the tensile curves and mechanical comparisons of the silk fibroin hydrogel were as shown in FIG. 10; before the temperature reached the glass transition temperature (77°C), the tensile properties of hydrogel scaffolds prepared at 25°C and 60°C had no obvious difference. After the temperature reached the glass transition temperature (77°C), the mechanical properties of hydrogel scaffolds prepared at 95°C were significantly higher than those of other groups of hydrogel scaffolds. However, when the temperature reached 130°C, due to the rapid loss of water molecules, the scaffolds ruptured when stretched to 60%, but the mechanical properties thereof decreased. The results show that coaxial hot stretching at a temperature above the glass transition temperature can significantly enhance the mechanical properties, whereas this effect decreases significantly after the glass transition temperature increases due to the loss of water molecules. Therefore, we determined 95°C as a preferable heating temperature.

### Embodiment 6 Effect of different stretching ratios of hot stretching on properties of silk fibroin hydrogel scaffold

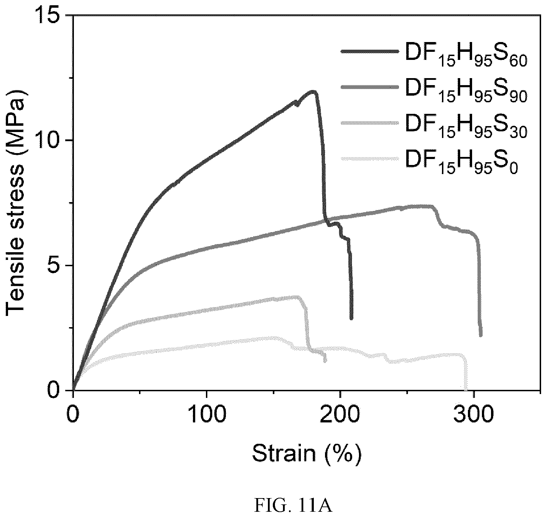

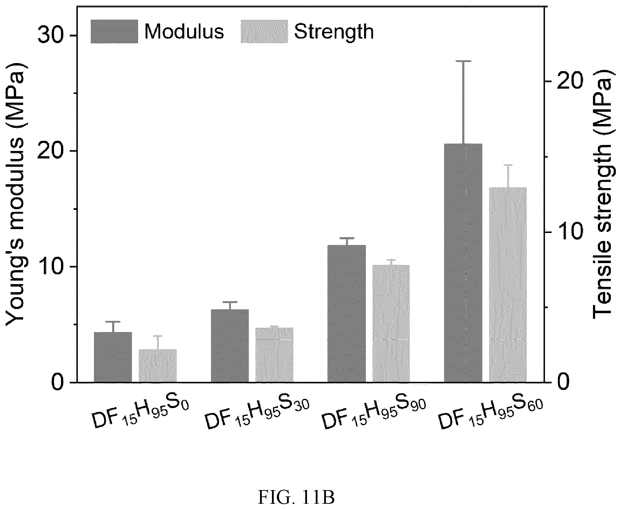

In this embodiment, a silk fibroin hydrogel was prepared by the method provided in Embodiment 1. In Step d, we set four stretching ratios; stretching was performed by using different stretching ratios (0%, 30%, 60% and 90%); the maximum tensile stress of the prepared hydrogel scaffold was tested and the mechanical properties thereof were tested by a biomechanical tester; the tensile curves and mechanical comparisons of the silk fibroin hydrogel were as shown in FIG. 11; when the stretching ratios increased from 0% to 60%, the mechanical properties of the scaffold gradually increased, indicating that the directional effect of the silk fibroin increases with the increased stretching ratios; when the stretching ratios further increased to 90%, the mechanical properties of the scaffold decreased, because the scaffold would be partially ruptured due to the excessive tensile ratio. Therefore, we determined 60% as a preferable tensile ratio.

### Embodiment 7 Synergistic effect of directional freezing and hot stretching on improvement in mechanical properties of materials

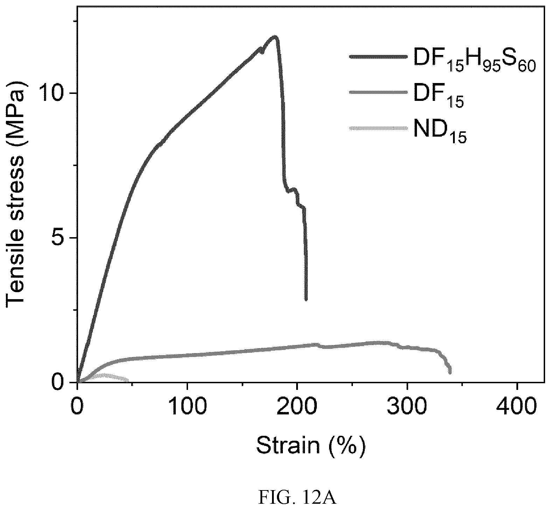

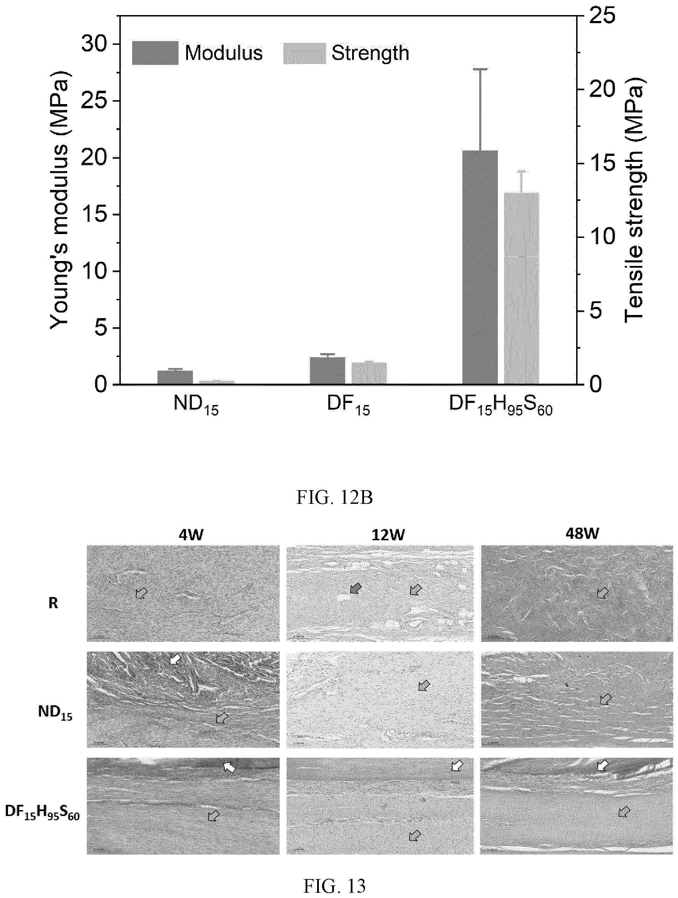

This embodiment relates to three groups of silk fibroin hydrogels. The silk fibroin hydrogels are prepared by the methods provided in Embodiment 1, Embodiment 2, and Embodiment 4 respectively, including ND₁₅, DF₁₅, and DF₁₅H₉₅S₆₀ hydrogels. FIG. 12 shows tensile stress-strain curves of three groups of hydrogels, and maximum tensile stresses and Young's moduli are statistically obtained. It can be concluded through comparison of ND₁₅ and DF₁₅ that on the premise that hot stretching is not performed, the maximum stresses and Young's moduli of the silk fibroin hydrogels can be increased respectively by about 6 times and 1 time through directional freezing. The DF₁₅ hydrogel was further subjected to hot stretching; as shown in FIG. 12, the maximum stress and Young's modulus of DF₁₅H₉₅S₆₀ increased by about 8 and 7 times respectively compared with those of the DF₁₅ hydrogel simply subjected to directional freezing, and by about 58 and 15 times respectively compared with those of the ND hydrogel.

In addition, the silk fibroin hydrogels are difficult to stretch but easy to tear when directly subjected to hot stretching without directional freezing or non-directional freezing.

Therefore, directional freezing and hot stretching have a synergistic effect on improvement in the mechanical properties of non-directional hydrogels.

### Example 8 Repair effect of silk fibroin hydrogel obtained under optimal conditions on rabbit tendon detachment model

DF₁₅H₉₅S₆₀ hydrogel has similar tensile strength and Young's modulus to tendons and can provide stable mechanical performance for a long time because of its slow in vivo degradation, and it is expected to be used as an artificial tendon substitute. To validate the potential of DF₁₅H₉₅S₆₀ hydrogel as a tendon patch, we transected the rabbit Achilles tendon and sutured the DF₁₅H₉₅S₆₀ hydrogel to the broken end of the tendon when tendon tension was kept, and the DF₁₅H₉₅S₆₀ hydrogel served as a lateral splint to provide mechanical compensation with a growth template. As shown in FIG. 13, at the early stage of repair (4 weeks), new tendons appeared in all groups (as indicated by a blue arrow), but new tendons in the detachment group were disorganized, with a larger number of rounded nuclei, more angiogenesis could be found, and some steatosis appeared (as indicated by a yellow arrow); whereas the new tendons in the DF₁₅H₉₅S₆₀ group were substantially arranged in parallel, sharing a direction with the DF₁₅H₉₅S₆₀ hydrogel; the nuclei was basically fusiform, and these morphologies were much more similar to those of normal tendons. In addition, it can be seen that there are only a very thin layer of fibrosis and an immunoreactive band around the DF₁₅H₉₅S₆₀ hydrogel, indicating the good biocompatibility of the DF₁₅H₉₅S₆₀ hydrogel. The DF₁₅H₉₅S₆₀ hydrogel itself shows little degradation, with high integrity (as indicated by a white arrow). The ND group had ruptures/dislocations due to the lack of adequate mechanical conditions and therefore could not provide effective mechanical support, and the new tendons were similar to those in the detachment group. At 12 weeks, the new tendons in the ND group and the detachment group appeared to be aligned in a large wave pattern, whereas the new tendons in the DF₁₅H₉₅S₆₀ group became further aligned. By the 48th week, the new tendons in the DF₁₅H₉₅S₆₀ group were very close to normal tendons and had no significant pathological differences from normal tendons.

In this application, these embodiments are also included as below.
1. A method for preparing a hydrogel, comprising the following steps:
   Step (1) : performing a directional freezing on a liquid solution including a first solvent and a silk fibroin as to make the solvent in the liquid solution change from a liquid state to a solid state, such obtaining a first product;
   step (2); enabling the solid solvent in the first product obtained in Step (1) to be sublimated from the solid state to a gaseous state, such obtaining a second product; and
   step (3) : performing directional hot stretching on the second product obtained in Step (2) to obtain the hydrogel.
2. The method according to clause 1, wherein the Step (2) further comprises cross-linking the silk fibroin .
3. The method according to one of clauses 1-2, wherein Step (2) further comprises solvating the cross-linked silk fibroin by a second solvent, wherein the second solvent is the same as the first solvent in the liquid solution in Step (1).
4. The method according to one of clauses 1-3, wherein the solvent comprises water.
5. The method according to one of clauses 1-4, wherein the solvent is pure water or mineral water.
6. The method according to one of clauses 1-5, wherein the directional of stretching is same as the direction of freezing.
7. The method according to one of clauses 1-6, wherein Step (1) comprises: providing a freezing source that enables the solvent in the solution to change from the liquid state to the solid state and enables the solution to approach the freezing source, such that the solution is directionally solidified from a direction approaching the freezing source to a direction departing from the freezing source.
8. The method according to one of clauses 1-7, wherein Step (1) further comprises pouring the solution into a chamber with an opening and performing the directional freezing on the solution in the chamber.
9. The method according to one of clauses 4-8, wherein a temperature in which the water changes from the liquid state to an a solid state is 0°C or below 0°C.
10. The method according to clause 9, wherein the temperature is below -20°C.
11. The n method according to one of clauses 1-10, wherein Step (1) further comprises providing a metal rod, wherein one end of the metal rod contacts with or is proximal to the solution in the chamber, while the other end of the metal rod contacts with a liquid nitrogen.
12. The preparation method according to one of clauses 8-11, wherein the chamber comprises a bottom, and a side of the metal rod covers the bottom of the chamber.
13. The preparation method according to one of clauses 3-12, wherein the cross-linking is chemical cross-linking or enzyme cross-linking.
14. The method according to one of clauses 1-13, wherein the sublimation in Step (2) comprises a freeze-drying step, and the freeze-drying step comprises:
   placing and freeze-drying the first product in step (1) in a freeze dryer as to make the ice to be the gaseous state, and then soaking the second product in step 2 into a anhydrous ethanol to be cross-linked, and finally soaking the cross-linked silk fibroin in the water to be fully hydrated; or
   directly cross-linking the second product in step 2, sublimating the solvent from the solid state to the gaseous state, and then soaking the sublimated solvent of the second product in the water to be fully hydrated.
15. The method according to one of clauses 1-14, wherein the directional hot stretching in Step (3) indicates heating up the second product in step (2) to above a glass transition temperature of the silk fibroin and then performing directional stretching on the second product.
16. The method according to one of clauses 1-15, wherein the glass transition temperature is below 80°C.
17. The method according to claim 16, wherein the hydrated second product is preheated at a temperature of 25°C to 130°C, and then the second product is stretched to 30% to 90% of an initial length.
18. A method, comprising:
   performing directional freezing on a solvent in a silk fibroin solution, such that the solvent changes from a liquid state to a solid state as to obtaining a first product;
   freeze-drying the solvent of the first product such that the solid-state solvent is sublimated as to obtaining a second product; and
   performing directional hot stretching on the second product,
   wherein a heating temperature is higher than a glass transition temperature of the second product.
19. The method according to clause 18, wherein the directional of the stretching is same as the direction of freezing.
20. The method according to clause 19, wherein the solvent is water.

The invention shown and described herein can be realized without any element or limitation specifically disclosed herein. Terms and expressions employed herein are used as descriptive terms and not as limitations, and it is not intended to exclude any equivalents of features shown and described or parts thereof in the use of these terms and expressions, and it should be recognized that various modifications are possible within the scope of the present invention. Therefore, it should be understood that although the present invention is specifically disclosed through various embodiments and optional features, modifications, and variations of the concepts described herein may be employed by a person skilled in the art and are considered to fall within the scope of the present invention as defined by the appended claims.

The contents of articles, patents, patent applications, and all other documents and electronically available information described or documented herein are incorporated herein by reference in their entirety to some extent, just as each individual publication is specifically and individually identified for reference. The applicant reserves the right to incorporate any and all materials and information from any such articles, patents, patent applications or other documents into this application.

## Claims

1. A method for preparing a hydrogel, comprising the following steps:
Step (1): performing a directional freezing on a liquid solution including a first solvent and a silk fibroin as to make the solvent in the liquid solution change from a liquid state to a solid state, such obtaining a first product;
step (2): enabling the solid solvent in the first product obtained in Step (1) to be sublimated from the solid state to a gaseous state, such obtaining a second product; and
step (3) : performing directional hot stretching on the second product obtained in Step (2) to obtain the hydrogel.

2. The method according to claim 1, wherein the Step (2) further comprises cross-linking the silk fibroin.

3. The method according to one of claims 1-2, wherein Step (2) further comprises solvating the cross-linked silk fibroin by a second solvent, wherein the second solvent is the same as the first solvent in the liquid solution in Step (1).

4. The method according to one of claims 1-3, wherein the solvent comprises water.

5. The method according to one of claims 1-4, wherein the solvent is pure water or mineral water.

6. The method according to one of claims 1-4, wherein the directional of stretching is same as the direction of freezing.

7. The method according to one of claims 1-6, wherein Step (1) comprises: providing a freezing source that enables the solvent in the solution to change from the liquid state to the solid state and enables the solution to approach the freezing source, such that the solution is directionally solidified from a direction approaching the freezing source to a direction departing from the freezing source.

8. The method according to one of claims 1-7, wherein Step (1) further comprises pouring the solution into a chamber with an opening and performing the directional freezing on the solution in the chamber.

9. The method according to one of claims 4-8, wherein a temperature in which the water changes from the liquid state to a solid state is 0°C or below 0°C.

10. The method according to claim 9, wherein the temperature is below -20°C.

11. The n method according to one of claims 1-10, wherein Step (1) further comprises providing a metal rod, wherein one end of the metal rod contacts with or is proximal to the solution in the chamber, while the other end of the metal rod contacts with a liquid nitrogen.

12. The preparation method according to one of claims 8-11, wherein the chamber comprises a bottom, and a side of the metal rod covers the bottom of the chamber.

13. The preparation method according to one of claims 2-12, wherein the cross-linking is chemical cross-linking or enzyme cross-linking.

14. The method according to claim 13, wherein the sublimation in Step (2) comprises a freeze-drying step, and the freeze-drying step comprises:
placing and freeze-drying the first product in step (1) in a freeze dryer as to make the ice to be the gaseous state, and then soaking the second product in step 2 into a anhydrous ethanol to be cross-linked, and finally soaking the cross-linked silk fibroin in the water to be fully hydrated; or
directly cross-linking the second product in step 2, sublimating the solvent from the solid state to the gaseous state, and then soaking the sublimated solvent of the second product in the water to be fully hydrated.

15. The method according to one of claims 1-14, wherein the directional hot stretching in Step (3) indicates heating up the second product in step (2) to above a glass transition temperature of the silk fibroin and then performing directional stretching on the second product.

16. Use of a silk fibroin hydrogel prepared by the method according to one of claims 1 to 15 in repair of ruptured tendons.
